# EUROPEAN PATENT SPECIFICATION

(11) **EP 0 859 579 B1**
(45) Date of publication and mention of the grant of the patent: **20.08.2003**
(21) Application number: 96936443.9
(22) Date of filing: 15.10.1996
(51) Int. Cl.: A61F 7/00, A47G 9/02

(54) **INFLATABLE BLANKET HAVING SELECTIVE AIRFLOW PATTERNS**
AUFBLASBARE DECKE MIT SELEKTIVEN LUFTSTRÖMUNGSMUSTERN
COUVERTURE GONFLABLE A PROFILS DE CIRCULATION DE L'AIR SELECTIFS

(30) Priority: 18.10.1995 US 544907; 18.10.1995 US 544501
(43) Date of publication of application: 26.08.1998
(73) Proprietor: MALLINCKRODT MEDICAL, INC., St. Louis, MO 63134 (US)
(72) Inventor: KAPPEL, Thomas, F., St. Louis, MO 63126 (US)
(74) Representative: Kruspig, Volkmar, Dipl.-Ing.
(86) International application number: US9616402
(87) International publication number: WO97014381

(56) References cited:
- EP-A- 0 311 336
- US-A- 4 660 388
- US-A- 5 097 548
- US-A- 5 304 213

## Description

Hypothermia is a condition of subnormal body temperature and presents serious consequences to the patient suffering therefrom. It has been shown that nearly seventy five percent of all patients who undergo surgical procedures develop hypothermia. This equates to approximately fourteen million patients a year in the United States alone. The hypothermic condition is brought on by many factors including anesthesia, the air conditioning of the operating room, and the infusion of cold blood, IV solutions, or irrigating fluids.

### 2. Background of the Related Art

Several methods and products have been developed to help prevent hypothermia from occurring, such as the use of infrared lamps, cotton blankets, and warm water mattresses. However, none of these methods and products have proven completely successful. In fact, it has been shown that these methods and products cannot even prevent the patients from losing their endogenous heat (*see, Journal of Post Anesthesia Nursing, 5(4)*:254-263, August 1990).

Another method of helping to prevent hypothermia that has proven very effective is the use of forced warm air convection. As early as 1937, a refrigeration blanket using cold air convection was suggested in U.S. Patent No. 2,093,834 to Gaugler. This blanket included a plurality of layers for channeling airflow from an inlet port. Non-inflatable portions were provided around the periphery of the blanket to secure the blanket around the body.

U.S. Patent No. 2,512,559 to Williams also relates to a blanket for providing cooled air to a person. The blanket in Williams comprised a plurality of thin sheets of material connected together at a plurality of discrete locations and connected together in a continuous line about the peripheral edge. An air inlet was provided to communicate with space between the sheets to allow cool air to be supplied thereto.

In U.S. Patent No. 4,572,188 to Augustine et al., a forced air convection system which can supply either cool or warm air to a blanket is described. The blanket in Augustine et al. comprises a plurality of inflatable hollow tubes having their interiors connected together through transverse openings. An entry port is provided in the upper surface of the blanket for admitting the cool or warm air and small exit ports are provided through the lower surface to allow the cool or warm air to flow out toward a body covered by the blanket.

Other patents relating to the supply of cool or warm air to a person through an inflatable blanket include U.S. Patent No. 4,660,388 to Greene, Jr.; No. 4,777,802 to Feher; No. 4,867,230 to Voss; No. 5,125,238 to Ragan et al.; No. 5,300,100 to Hickle et al.; No. 5,300,102 to Augustine et al.; No. 5,324,320 to Augustine et al.; No. 5,343,579 to Dickerhoff et al.; No. 5,360,439 to Dickerhoff et al.; and No. 5,384,924 to Dickerhoff et al. Each of these patents describe blankets having various attributes and configurations to supply cool or warm air to the person.

An inflatable blanket according to the preamble of claim 1 is known from both documents US-A-4 660 388 and EP-A-0 311 336.

While there are a number of patents noted above and others not mentioned which relate to inflatable blankets for use in supplying cool or warm air to a patient, there remains a need in the art for improvements to forced air convection systems.

### OBJECTS OF THE INVENTION

It is one object of the present invention to provide a blanket for a forced air convection system which allows air to be directed only toward the patient, and not to areas around the patient.

It is another object of the present invention to provide a blanket for a forced air convection system which includes means for selectively providing air to portions of a patient's body.

It is a further object of the present invention to provide a blanket for a forced air convection system which allows the greatest transfer of heated or cooled air to be directed to selected portions of the patient.

### SUMMARY OF THE INVENTION

The above objects and others are accomplished according to the present invention by providing a blanket for a forced air convective system which includes exit perforations arranged in selective patterns and/or having different sizes.

### BRIEF DESCRIPTION OF THE DRAWINGS

Fig. 1 is a plan view of a blanket for a forced air convection system according to one embodiment of the present invention.
Fig. 2 is a plan view of a blanket for a forced air convection system according to a further embodiment of the present invention.
Fig. 3 is a plan view of a blanket for a forced air convection system according to one embodiment of the present invention.
Fig. 4 is a schematic side view, with portions broken away, of a blanket in accordance with the invention.

### DETAILED DESCRIPTION OF THE PREFERRED EMBODIMENTS

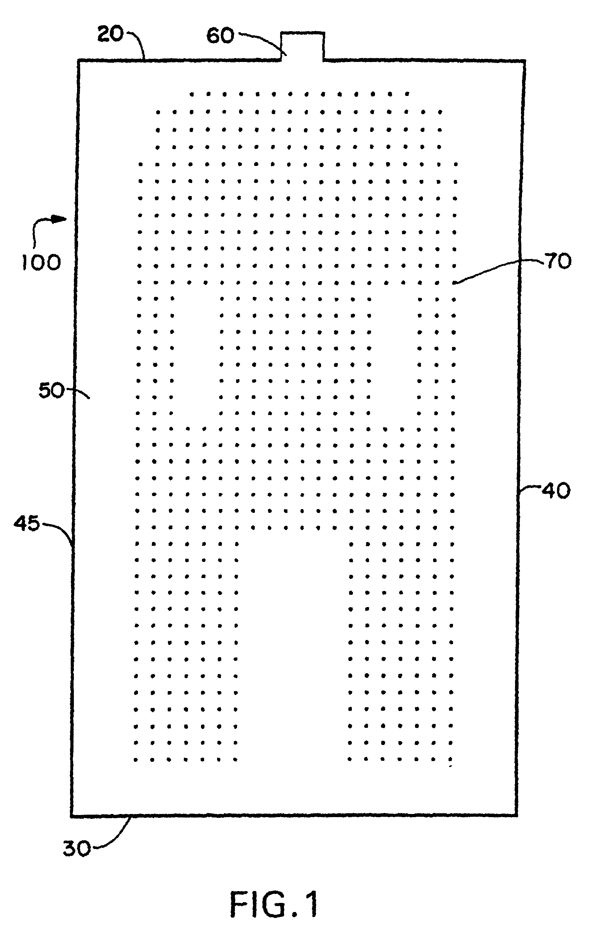

Fig. 1 is a plan view of a blanket, generally designated by reference numeral 10, for a forced air convection system, according to one embodiment of the present invention. In particular, blanket 10 includes an upper or head end 20, a lower or foot end 30, and two sides 40, 45. The blanket 10 further includes an upper sheet of material (not visible), and a lower sheet of material 50. The upper sheet and lower sheet 50 are sealed together around respective peripheral edges to form a cavity therebetween, which may be inflated by introduction of air from an appropriate source. The upper sheet and lower sheet 50 may further be connected together in any one of several desirable configurations, such as spot welds, interconnected columns, interconnected tubes, etc. The blanket 10 includes at least one inlet port 60 for attachment to a source of forced air which will be used to inflate the blanket 10, and provide either warming or cooling air to the patient. As shown in Fig. 1, the inlet port 60 is formed along one end of the blanket 10. However, other configurations are equally acceptable and are within the scope of the present invention, as will be further discussed below. The lower sheet of the blanket 10 includes a plurality of perforations or small exit holes 70 formed therethrough which allow air to escape from the blanket 10 toward a patient.

In order to provide heated or cooled air selectively only to the patient, the perforations 70 are provided in a selective pattern through the lower sheet 50. In particular, as shown in Fig. 1, the perforations 70 are arranged in the general shape of a patient's body.

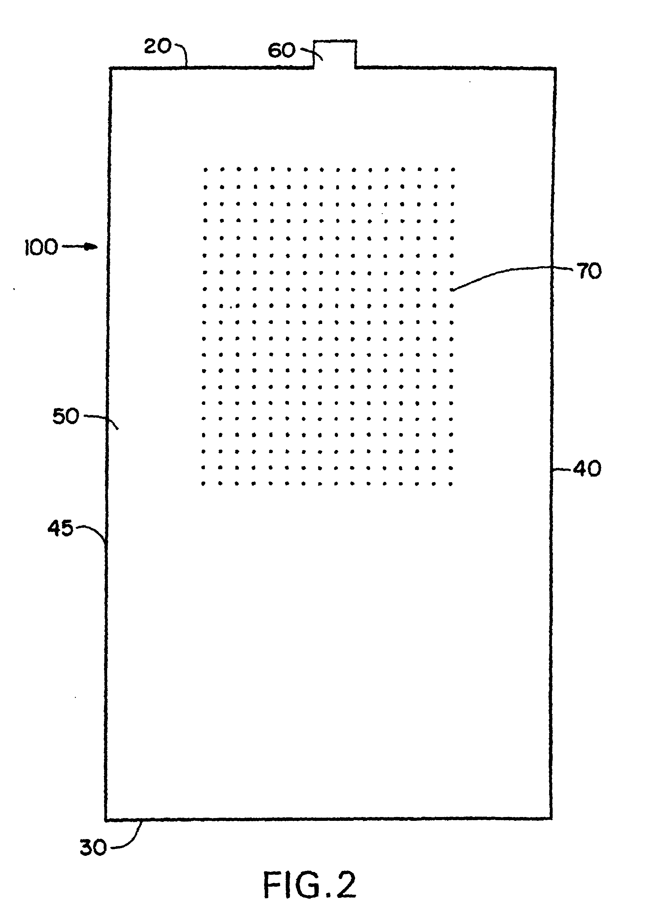

In addition, it is often desirable to provide heated or cooled air to a selective portion of a patient. Therefore, as shown in Fig. 2, the perforations 70 are provided in a limited, and selected pattern through the lower sheet 50. In particular, as shown in Fig. 2, the perforations 70, are provided only in an area which will cover the chest of a patient.

The present invention relates to any number of patterns of perforations. In addition, perforations may be provided only over one area or may be provided over multiple areas for selectively providing heated or cooled air to directly to those areas of a patient which the perforations cover.

By providing perforations only through selective areas of the lower sheet of the blanket, it is possible to provide heating or cooling to a patient in a more efficient manner. In particular, when perforations are provided over the entire surface area of the lower sheet, air provided through perforations not directly covering a patient may not add significantly to the heating or cooling of the patient. Providing selectively patterned perforations in accordance with the present invention assures that the greatest amount of heating or cooling air will be provided directly to the patient.

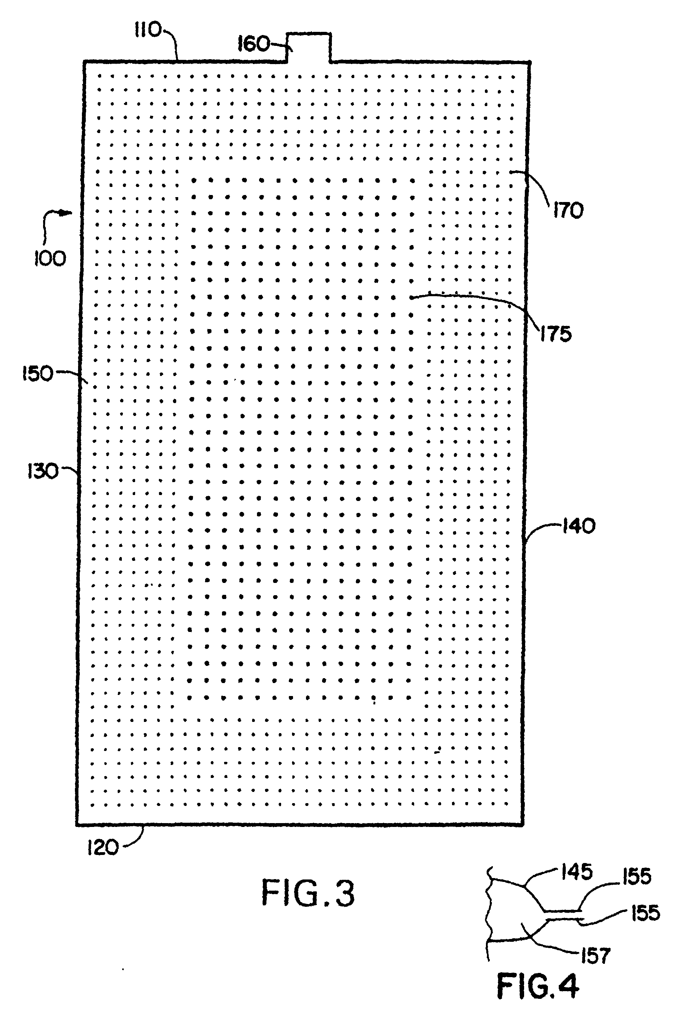

Fig. 3 is a plan view of a blanket, generally designated by reference numeral 100, for a forced air convection system, according to one embodiment of the present invention. In particular, blanket 100 includes an upper or head end 110, a lower or foot end 120, and two sides 130, 140. The blanket 100 further includes an upper sheet of material 145 and a lower sheet of material 150. The upper sheet and lower sheets 145, 150 are sealed together around respective peripheral edges 155 to form a cavity 157 therebetween, which may be inflated by introduction of air from an appropriate source. The upper sheet and lower sheets 145, 150 may further be connected together in any one of several desirable configurations, such as spot welds, interconnected columns, interconnected tubes, etc. The blanket 100 includes at least one inlet port 160, for attachment to a source of forced air which will be used to inflate the blanket 100, and provide either warming or cooling air to the patient. As shown in Fig. 1, the inlet port 160 is formed along one end of the blanket 100. However, other configurations are equally acceptable and are within the scope of the present invention, as will be further discussed below. The lower sheet of the blanket 100 includes a plurality of perforations or small exit holes 170, 175 formed therethrough which allow air to escape from the blanket 100 toward a patient.

It is desirable to provide the greatest transfer of heated or cooled area to the trunk of the patient. Therefore, as shown in Fig. 1, the perforations 175, provided over a middle portion of the blanket 100, have a larger size than perforations 170, provided in peripheral areas of the blanket 100. In one particular embodiment of the present invention, the larger perforations 175 are arranged in the general shape of a patient's body.

The large perforations according to the present invention preferably have a diameter in the range of (.075 to .150 inches)
.191 to .381 centimeters. The small perforations according to the present invention preferably have a diameter in the range of (.058 to .116 inches)
.147 to .295 centimeters.

The present invention relates to any number of patterns of large and small perforations. For example, the large perforations may be provided only over the chest area of a patient, or over a particular area of a patient to which it is desired to provide greater transfer of heated or cooled air. In addition, the large perforations may be provided in more than one area, to produce additional areas of more efficient transfer of heated or cooled air.

In addition, more than two sizes of perforations may be provided. The largest perforations would be used in those areas where the greatest transfer of heated or cooled air is desired, and the smallest perforations would be used in those areas where the least transfer of heated or cooled air is desired. Perforations having an intermediate size or several intermediate sizes can be provided in other areas of the blanket. In particular, this intermediate perforations may be provided in areas between the large and small perforations so as to create a gradient of air transfer across the blanket.

By providing the pattern of large and small perforations, it is possible to provide hearing or cooling to a patient in a more efficient manner. In particular, the large perforations provide greater transfer of heated or cooled air directly to the patient, while small perforations are used in the peripheral areas of the blanket not directly covering a patient. Providing the patterned large and small perforations in accordance with the present invention assures that the greatest amount of heating or cooling air will be provided directly to the patient.

The blankets shown in Figs. 1, 2 and 3 represent full body blankets, but the present invention would be equally applicable to blankets intended to cover only portions of the patient, such as an upper body blanket or a lower body blanket. The blankets according to the present invention are also equally useful in both adult and pediatric sizes. Additionally, the blankets according to the present invention may be used equally effectively in either the operating room or in other areas of the hospital, such as the PACU. Moreover, as noted, the blankets according to the present invention may be used to provide either warming or cooling to a patient.

As noted above, the inlet port as shown in Figs. 1 and 2 is located at a corner of the blanket. However, the inlet port may be located at almost any position which allows the blanket to be easily inflated. For example, the inlet port may be provided along any edge of the blanket or through the upper or lower sheet of the blanket at a location spaced away from the edge of the blanket. In addition, multiple inlet ports may be provided to increase the versatility of the blanket.

The blankets of the present invention may be formed of any suitable material capable of being sealed together at selected positions and having sufficient strength to allow inflation and adequate air distribution within the inflated portion. Such materials include plastics, non-woven wood pulp compositions, laminated plastic and wood pulp materials, and combinations thereof.

The foregoing has been a description of certain preferred embodiments of the present invention, but is not intended to limit the invention in any way. Rather, many modifications, variations and changes in details may be made within the scope of the present invention.

## Claims

1. An Inflatable blanket (100) for a forced air convection system comprising:
- an upper sheet (145) of material having a generally rectangular shape with an upper end (110), a lower end (120) and two sides (130, 140);
- a lower sheet (150) of material having a generally rectangular shape with an upper end(110), a lower end (120) and two sides (130, 140);
wherein said upper sheet (145) and said lower sheet (150) are sealed together around their peripheral edges (155) at their respective upper ends (110), lower ends (120) and sides (130, 140), to create a full body blanket with an inflatable cavity (157) having an upper end, a lower end and two sides therebetween;
- an inflating port (160) connecting said inflatable cavity (157) with the atmosphere and through which inflation medium may be introduced to said inflatable cavity (157) to inflate said blanket (100); and
- a plurality of perforations (170) formed through said lower sheet (150),
**characterized in that**, said plurality of perforations (170) are provided as a first area of large perforations (170) in said lower sheet (150), which first area is at least partly surrounded by a plurality of smaller perforations (175).

2. A blanket according to claim 1,
**characterized in that**, said plurality of perforations (170) are arranged in a specific pattern to allow air to exit only toward selective portions of a patient's body,
wherein the specific pattern has a general shape of a patient's body or of said selective portions thereof.

3. A blanket according to claim 2,
**characterized in that**,
the specific pattern has a shape which will cover a patient's chest.

4. A blanket according to claim 1,
**characterized in that**,
said large perforations (170) are provided in said first area for positioning directly over a patient's body and said small perforations (175) are provided In areas peripheral to the first area where said larger perforations (170) are provided.

5. A blanket according to claim 1,
**characterized in that**,
said large perforations (170) are provided In said first area for covering a chest of a patient and said small perforations (175) are provided in areas peripheral to the first area where said larger perforations (170) are provided.

6. A blanket according to anyone of the claims 1, 4, 5,
**characterized in that**,
said large perforations (170) have a diameter in the range of 0,191 to 0,381 cm (.075 to .150 inches) and said small perforations (175) have a diameter in the range of 0,147 to 0,295 cm (.058 to .116 inches).

7. A blanket according to anyone of the claims 1, 4, 5, 6,
**characterized in that**,
said large perforations (170) are provided in more than one area of said blanket (100) and small perforations (175) are provided in areas peripheral to said large perforation areas.

8. A blanket according to anyone of the preceeding claims,
**characterized in that**,
said perforations (170, 175) are provided in three or more different sizes.

9. A blanket according to claim 8,
**characterized in that**,
said perforations (170, 175) of different sizes are arranged in a pattern which provides a gradient of air transfer across said blanket (100).

10. A blanket according to anyone of the claims 1, 4 to 9,
**characterized in that**,
said first area of large perforations (170) is completely surrounded by said small perforations (175).

## Patentansprüche

1. Aufblasbare Decke (100) für ein Druckluft-Konvektionssystem mit:
- einer oberen Lage (145) eines Materials einer allgemein rechtwinkligen Form mit einem oberen Ende (110), einem unteren Ende (120) und zwei Seiten (130, 140);
- einer unteren Lage (150) eines Materials einer allgemein rechtwinkligen Form mit einem oberen Ende (110), einem unteren Ende (120) und zwei Seiten (130, 140); wobei die obere Lage (145) und die untere Lage (150) um ihre Umfangskanten an ihren jeweiligen oberen Enden (110), unteren Enden (120) und Seiten (130, 140) miteinander verschweißt sind, um dazwischen eine Ganzkörperdecke mit einem aufblasbaren Hohlraum (157) mit einem oberen Ende (110), einem unteren Ende (120) und Seiten (130, 140) zu schaffen;
- einer Aufblasöffnung (160), die den aufblasbaren Hohlraum (157) mit der Atmosphäre verbindet, und durch den ein Aufblasmedium in den aufblasbaren Hohlraum (157) eingeleitet werden kann, um die Decke (100) aufzublasen; und
- einer Vielzahl durch die untere Lage (150) ausgeführter Perforierungen (170),
**dadurch gekennzeichnet, dass**
die Vielzahl Perforierungen (170) als ein erster Bereich mit großen Perforierungen (170) in der unteren Lage (150) angeordnet ist, wobei der erste Bereich zumindest teilweise von einer Vielzahl kleiner Perforierungen (175) umgeben ist.

2. Decke nach Anspruch 1,
**dadurch gekennzeichnet, dass**
die Vielzahl Perforierungen (170) in einem bestimmten Muster angeordnet ist, damit Luft nur in Richtung ausgewählter Abschnitte des Körpers eines Patienten austreten kann,
wobei das bestimmte Muster die allgemeine Form des Körpers eines Patienten oder der ausgewählten Abschnitte davon hat.

3. Decke nach Anspruch 2,
**dadurch gekennzeichnet, dass**
das bestimmte Muster eine Form hat, die die Brust eines Patienten bedeckt.

4. Decke nach Anspruch 1,
**dadurch gekennzeichnet, dass**
die großen Perforierungen (170) im ersten Bereich zur Positionierung direkt über dem Körper eines Patienten angeordnet sind und die kleinen Perforierungen (175) in peripheren Bereichen des ersten Bereichs mit den großen Perforierungen (170) vorgesehen sind.

5. Decke nach Anspruch 1,
**dadurch gekennzeichnet, dass**
die großen Perforierungen (170) im ersten Bereich zur Abdeckung der Brust eines Patienten angeordnet sind und die kleinen Perforierungen (175) in peripheren Bereichen des ersten Bereichs mit den großen Perforierungen (170) vorgesehen sind.

6. Decke nach einem der Ansprüche 1, 4, 5,
**dadurch gekennzeichnet, dass**
die großen Perforierungen (170) einen Durchmesser im Bereich von 0,191 bis 0,381 cm (0,075 bis 0,150 Zoll) und die kleinen Perforierungen einen Durchmesser im Bereich von 0,147 bis 0,295 cm (0,058 bis 0,116 Zoll) haben.

7. Decke nach einem der Ansprüche 1, 4, 5, 6,
**dadurch gekennzeichnet, dass**
die großen Perforierungen (170) in mehr als einem Bereich der Decke (100) und
die kleinen Perforierungen (175) in peripheren Bereichen der Bereiche mit den großen Perforierungen vorgesehen sind.

8. Decke nach einem der vorhergehenden Ansprüche,
**dadurch gekennzeichnet, dass**
die Perforierungen (170, 175) in drei oder mehr verschiedenen Größen vorgesehen sind.

9. Decke nach Anspruch 8,
**dadurch gekennzeichnet, dass**
die Perforierungen (170, 175) verschiedener Größen in einem Muster angeordnet sind, das ein Luftströmungsgefälle über die Decke (100) bereitstellt.

10. Decke nach einem der Ansprüche 1, 4 bis 9,
**dadurch gekennzeichnet, dass**
der erste Bereich mit großen Perforierungen (170) vollständig von den kleinen Perforierungen (175) umgeben ist.

## Revendications

1. Couverture gonflable (100) pour un système de convection d'air forcé, comprenant :
-- une feuille supérieure (145) d'un matériau ayant une forme généralement rectangulaire avec une extrémité supérieure (110), une extrémité inférieure (120) et deux côtés (130, 140) ;
-- une feuille inférieure (150) d'un matériau ayant une forme généralement rectangulaire avec une extrémité supérieure (110), une extrémité inférieure (120) et deux côtés (130, 140) ;
dans laquelle ladite feuille supérieure (145) et ladite feuille inférieure (150) sont soudées ensemble autour de leur bordure périphérique (155) au niveau de leurs extrémités supérieures respectives (110), de leurs extrémités inférieures respectives (120), et de leurs côtés respectifs (130, 140) afin de créer une couverture corporelle totale avec une cavité gonflable (157) ayant une extrémité supérieure, une extrémité inférieure, et deux côtés entre ces extrémités ;
-- un orifice de gonflage (160) qui connecte ladite cavité gonflable (157) avec l'atmosphère et à travers lequel un fluide de gonflage peut être introduit dans ladite cavité gonflable (157) pour gonfler ladite couverture (100) ; et
-- une pluralité de perforations (170) formées à travers ladite feuille inférieure (150), **caractérisée en ce que**
ladite pluralité de perforations (170) sont prévues sous forme d'une première zone de grandes perforations (170) dans ladite feuille inférieure (150), ladite première zone étant au moins partiellement entourée par une pluralité de perforations plus petites (175).

2. Couverture selon la revendication 1, **caractérisée en ce que** :
ladite pluralité de perforations (170) sont agencées suivant un motif spécifique pour permettre à l'air de sortir uniquement vers des portions sélectives du corps d'un patient,
ledit motif spécifique ayant la forme générale du corps d'un patient, ou desdites portions sélectives de celui-ci.

3. Couverture selon la revendication 2, **caractérisée en ce que** ledit motif spécifique a une forme qui va couvrir le torse d'un patient.

4. Couverture selon la revendication 1, **caractérisée en ce que** lesdites grandes perforations (170) sont prévues dans ladite première zone destinée à être positionnée directement au-dessus du corps d'un patient, et lesdites petites perforations (175) sont prévues dans des zones périphériques à la première zone dans laquelle sont prévues lesdites grandes perforations (170).

5. Couverture selon la revendication 1, **caractérisée en ce que** lesdites grandes perforations (170) sont prévues dans ladite première zone pour couvrir le torse d'un patient, et lesdites petites perforations (175) sont prévus dans des zones périphériques à la première zone dans laquelle sont prévues lesdites grandes perforations (170).

6. Couverture selon l'une quelconque des revendications 1, 4 et 5, **caractérisée en ce que** lesdites grandes perforations (170) ont un diamètre dans la plage de 0,191 à 0,381 cm (0,075 à 0,150 pouces) et lesdites petites perforations (175) ont un diamètre dans la plage de 0,147 à 0,295 cm (0,058 à 0,116 pouces).

7. Couverture selon l'une quelconque des revendications 1, quatre, cinq et 6, **caractérisée en ce que** lesdites grandes perforations (170) sont prévues dans plus d'une zone de ladite couverture (100), et **en ce que** lesdites petites perforations (175) sont prévues dans des zones périphériques auxdites zones de grandes perforations.

8. Couverture selon l'une quelconque des revendications précédentes, **caractérisée en ce que** lesdites perforations (170, 175) sont prévues en trois tailles différentes ou plus.

9. Couverture selon la revendication 8, **caractérisé en ce que** lesdites perforations (170, 175) de tailles différentes sont agencées sous un motif qui assure un gradient de transfert d'air à travers ladite couverture (100).

10. Couverture selon l'une quelconque des revendications 1 et 4 à 9, **caractérisée en ce que** ladite première zone de grandes perforations (170) est complètement entourée par lesdites petites perforations (175).
